# EUROPEAN PATENT SPECIFICATION

(11) **EP 3 968 873 B1**
(45) Date of publication and mention of the grant of the patent: **13.12.2023**
(21) Application number: 20726552.1
(22) Date of filing: 10.05.2020
(51) Int. Cl.: A61B 17/16

(54) **BONE PUNCH INSTRUMENT FOR CREATING A BONE TUNNEL**
KNOCHENSTANZENINSTRUMENT ZUR HERSTELLUNG EINES KNOCHENKANALS
INSTRUMENT DE PERFORATION OSSEUSE DE CRÉATION D'UN TUNNEL OSSEUX

(30) Priority: 14.05.2019 US 201916411879
(43) Date of publication of application: 23.03.2022
(73) Proprietor: Eggli, Stefan, 1723 Pierrafortscha (CH)
(72) Inventor: HÄBERLI, Janosch, 3012 Bern (CH); EGGLI, Stefan, 1723 Pierrafortscha (CH); OVERES, Tom, 4536 Attiswil (CH)
(74) Representative: Vesterinen, Jussi Tapio
(86) International application number: PCT/IB2020/054410
(87) International publication number: WO 2020/229988

(56) References cited:
- JP-A- 2008 295 901
- US-A- 5 885 293
- US-A1- 2007 135 924
- US-A1- 2014 236 306
- US-A1- 2016 151 076

## Description

### TECHNICAL FIELD OF THE INVENTION

The present invention relates to a bone punch instrument for creating a bone tunnel in a target bone, such as the lateral or medial femoral condyle. The invention also relates to a bone punch instrument kit that can be used to create the bone tunnel.

### BACKGROUND OF THE INVENTION

In orthopedic interventions around the knee, bone plugs are often retrieved from a harvesting site and transferred to a target site. An example surgical intervention process for such a transplantation is the anterior cruciate ligament (ACL) reconstruction process, during which autologous tissues, such as tendons and ligaments, and bone blocks / plugs are used. One example technique for ACL reconstruction uses the quadriceps tendon and a patella bone plug in the reconstruction process. This arthroscopically performed surgery includes the following procedures.

### Procedure 1: Quadriceps tendon graft harvesting

A central piece of the quadriceps tendon graft having a length of e.g. approximately 5 cm including an attached proximal patellar bone plug having a length of e.g. approximately 2 cm is harvested. This graft will serve as a replacement for the torn anterior cruciate ligament.

### Procedure 2: Tibial tunnel creation

A tunnel is drilled from the anterior and proximal tibia toward the natural tibial foot-print of the ACL. Preferably a hollow drill is used in order to retrieve a bone plug, which can be placed back in the tibial tunnel, at a later step during the surgery. The tibial tunnel is shaped as a through bore having of a diameter of approximately 10 mm.

### Procedure 3: Femoral tunnel preparation

A guide wire is placed through the femoral bone, defining the intended anatomical direction of the femoral tunnel. Using a cannulated drill, the guide wire is over-drilled and the tunnel is created. The femoral tunnel is shaped as a blind hole having a diameter of approximately 8 mm and a length of approximately 25 mm.

### Procedure 4: Graft placement / ACL reconstruction

The patellar bone plug including the quadriceps tendon as retrieved in procedure 1 is impacted into the femoral tunnel. The quadriceps graft is pulled over the joint space, and fixated in the tibial tunnel. Fixation may be carried out using a screw for suture attachment or an interference screw. As a next step, the tibial tunnel is filled with the bone plug, obtained when creating the tibial tunnel.

As described, normally the femoral tunnel is created using a cannulated drill, guided by the femoral guide wire. By means of drilling, the bone is removed. The disadvantage of this technique is that drilling implicitly creates drilling debris. This bone debris is captured within the joint space and forms swimming bone sediments in the synovial joint fluid.

Several disadvantages are associated with these sediments / bone debris:
- The joint needs to be washed thoroughly during the surgery, which prolongs surgery time.
- Remaining residues need to be built down by the body.
- The sediments drastically reduce the camera view during the arthroscopically performed surgery.
- Good bone is lost, which could be used to fill the patella graft site, and promote healing.

US2016151076A1 discloses a drill tool for implant surgery including a first drill part having a first, smaller diameter for drilling a recess for an implant post and a second drill part having a second, larger diameter for drilling a recess for an implant hat. The second drill part has one or more shape cutting edges and one or more sharp precutting edges extending beyond said one or more shape cutting edges.

US2014236306A1 discloses techniques and instruments for knee replacement surgery that allow for the removal of an oval oblong-shaped allograft bone and cartilage plug from a donor distal femur. The instruments include (i) sizing guides to match the recipient's femoral size and curvature to that of a donor femur (the sizing guides also acting as a wide pin placement template for the donor distal femur); (ii) osteotomes that cut the curved and straight portions of the implant shape (these may be disposable or reusable); and (iii) templates that fit over the guide pins and have openings to allow the osteotomes to cut the donor femur plug to the correct size, shape and depth. The instruments allow for a non-circular shape to be extracted from a donor femur for use in a bone-saving osteoarthritis distal femur resurfacing procedure.

### SUMMARY OF THE INVENTION

It is an object of the present invention to overcome at least some of the problems associated with bone tunnel creation instruments and techniques, which may include femoral tunnel creation techniques.

According to a first aspect of the invention, there is provided a femoral bone punch instrument as recited in claim 1.

By using the punch instrument comprising an impaction handle and a punch head, a bone plug can be retrieved without creating bone drilling debris. Furthermore, the bone plug can be retrieved as a solid piece of graft, which may be used to augment another site in the body, and therefore to promote healing and to reduce recovery time after surgery. The proposed instrument may be used e.g. for creating a bone tunnel in a lateral femoral condyle for anterior cruciate ligament reconstruction surgery or for creating a bone tunnel in a medial femoral condyle for posterior cruciate ligament reconstruction surgery. The proposed bone punch instrument has further the advantage that thanks to the modular structure of the bone punch instrument, the impaction handle may be used many times, and not necessarily with the punch head to create bone tunnels. The punch head may be changed every time when a bone tunnel is created. In other words, the punch head may be a single-use device, i.e. disposable, while the impaction handle may be a multiple-use device. In this manner, a cutting edge of the punch head is always sharp and it can be ensured that the punch head is sterile.

The bone punch instrument may further comprise a longitudinal, cannulated centralization element sized and shaped to be at least partially received in the punch head bore.

The centralization element may be longitudinally divided into a first centralization element section characterized by a first centralization element outer diameter, and a second centralization element section characterized by a second centralization element outer diameter, which is smaller than the first centralization element outer diameter, and wherein the first centralization element section is sized and shaped to be slidingly and in a form-fit manner received within the punch head bore, and the second centralization element section is sized and shaped to be at least partially slidingly and in a form-fit manner received within a cannulation of the impaction handle.

The centralization element may be longitudinally divided into a first centralization element section characterized by a first centralization element outer diameter, and a second centralization element section characterized by a second centralization element outer diameter, which is smaller than the first centralization element outer diameter, wherein the first centralization element section is oversized in relation to a punch inner diameter, and comprises at least one longitudinal slot providing elasticity and a friction fit when inserted into the punch head and/or moving within the punch head.

The bone punch instrument may further comprise a guide wire, wherein the centralization element is configured to be placed around the guide wire, and the centralization element is sized and shaped to be at least partly received in the punch head bore for centralizing the punch head around the guide wire.

Openings and/or protrusions may be provided on an inner surface of the wall facing the punch head bore.

The bone punch instrument may further comprise a punch head holder comprising a punch head holder bore sized and shaped to at least partially receive the punch head and the bone plug, the punch head holder bore comprising a stop element for preventing the punch head from moving beyond the stop element while allowing the bone plug to move beyond the stop element to enable removal of the bone plug out of the punch head.

The punch head may comprise at least one first key-face forming a first geometry, wherein the bone punch instrument may further comprise a punch head holder comprising a punch head receiving pocket sized and shaped for at least partially receiving the punch head, wherein the punch head holder comprises at least one second key-face forming a second geometry, and wherein upon engagement of the first and second geometries, a rotational degree of freedom between the punch head and the punch head holder is blocked.

According to a second aspect of the invention, there is provided a bone punch instrument kit as recited in claim 13.

By changing the size of a punch head used in the process, the shape and/or size of the bone plug can be chosen as desired. Thus, the bone punch instrument may be used for target bones of various sizes.

Other aspects of the invention are recited in the dependent claims attached hereto.

### BRIEF DESCRIPTION OF THE DRAWINGS

Other features and advantages of the invention will become apparent from the following description of a non-limiting example embodiment, with reference to the appended drawings, in which:
- Figure 1 depicts a bone punch instrument in an exploded view;
- Figures 2A and 2B depict a handle of the bone punch instrument of Figure 1 in a perspective view and a cross-sectional view, respectively;
- Figures 3A and 3B depict a punch head of the bone punch instrument of Figure 1 in a perspective view and a cross-sectional view, respectively;
- Figures 4A and 4B depict a centralization element of the bone punch instrument of Figure 1 in a perspective view and a cross-sectional view, respectively;
- Figure 4C depicts another example centralization element in a perspective view;
- Figures 5A to 5H depict the process of creating a femoral tunnel;
- Figure 6 depicts a flow chart summarizing the method steps of creating a femoral bone tunnel;
- Figures 7A to 7D depict in perspective views a punch head holder for receiving the punch head for removing a bone plug out of the punch head during different phases of the bone plug removal process; and
- Figures 8A to 8C depict in perspective views another example punch head holder for separating an impaction handle from a punch head.

### DETAILED DESCRIPTION OF THE INVENTION

An embodiment of the present invention will now be described in detail with reference to the attached figures. This embodiment is described in the context of creating a hole in the femoral bone, and more specifically in the lateral femoral condyle, while at the same time allowing a bone graft element to be retrieved out of the created hole, but the teachings of the invention are not limited to this environment. The teachings of the present invention are equally applicable for creating holes in other bones as well. Identical or corresponding functional and structural elements which appear in the different drawings are assigned the same reference numerals.

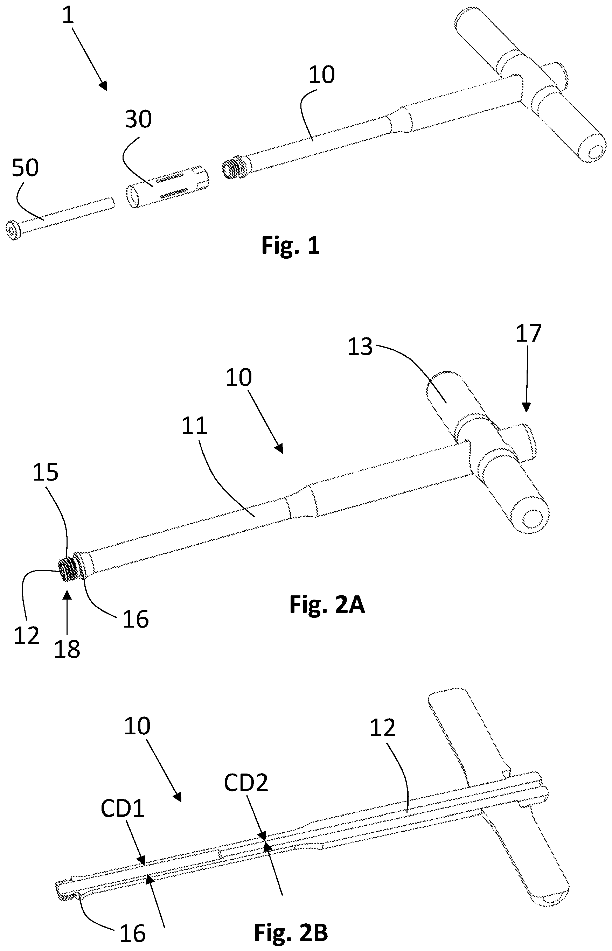

Figure 1 shows the bone punch assembly, instrument or instrumentation 1 in an exploded view. In this example embodiment, the punching instrument comprises three physically separate elements, which may be connected or coupled to each other as explained later in more detail. As shown in Figure 1, the interacting elements or instruments are an impaction handle or element 10, a punch head or element 30, and a centralization aid or element 50. It is to be noted that the centralization element is optional. Furthermore, according to one variant, the impaction handle and the punch head could be one single monobloc element, i.e. constructed from a single piece.

Figures 2A and 2B depict the impaction handle in a perspective view and a cross-sectional view, respectively. The impaction handle 10 comprises an elongated shaft 11 (defining a longitudinal axis, which may follow a straight or curved imaginary line) including a cannulation or channel 12 that extends through the shaft between a first end or extremity, referred to as a force receiving end or a proximal end 17, and a second end, referred to as a punch head coupling end or a distal end 18. In the present description, a proximal end refers to the end, which is closer to a user operating the instrument as it is intended to be used, whereas the distal end refers to the end away from the user. In the present embodiment, the impaction handle is a T-handle, comprising a grip element 13 which is oriented substantially perpendicular to the shaft. The grip element may in fact be manufactured as a physically separate element from the shaft so that the grip element, which is a straight or curved bar, is then firmly coupled (by e.g. welding, gluing and/or friction, etc.) to the shaft to form the impaction handle. In the present embodiment, the grip element has a through hole such that the shaft passes through the hole. As shown in Figures 2A and 2B, the hole axis is orthogonal or substantially orthogonal to the longitudinal axis of the grip element 13. Alternatively, the impaction handle can be a straight or bent element, etc. A T-handle provides the advantage to comfortably be able to exert a torque on the target bone, to break off a bone plug as described in greater detail later.

The impaction handle comprises at its distal end 18 a coupling feature or coupling means for coupling the impaction handle 10 to the punch head 30. The coupling means in this embodiment comprise a first connection or coupling element, and more specifically a first thread 15. However, a snap element or another friction connection element could be used instead. As can be seen e.g. in Figure 2A, the first thread is in this example a male thread formed on the outer surface of the impaction handle 10. The first thread is shaped and sized to engage with a second thread 34 formed on the punch head 30. More specifically, the second thread is a female thread formed on the inner surface of the punch head at its proximal end 17. The distal end 18 of the impaction handle further comprises a seat 16 or a stop element sized and shaped to mate against the punch head 30 and more specifically the proximal end of the punch head 30. The seat forms an assembly limit and a force transfer area between the punch head and the impaction handle.

As shown in Figure 2B, the cannulation 12 is a stepped cannulation, having more than one diameter. More specifically, the cannulation 12 comprises a first cannulation section having a first cannulation diameter CD1, and a second cannulation section having a second cannulation diameter CD2. In the present description when referring to a diameter of an object, the object does not necessarily have a circular cross section. The first cannulation section extends from the distal end toward the proximal end, while the second cannulation section extends from the proximal end toward the distal end. In this example, CD1 is greater than CD2. The first cannulation section is shaped and sized to receive in a substantially form fit manner and slidingly a portion of the centralization element as explained later in more detail. The second cannulation section is shaped and sized to receive in a substantially form fit manner and slidingly a guide wire as explained later in more detail. Alternatively, the cannulation 12 can have one single diameter or more than two cannulation diameters.

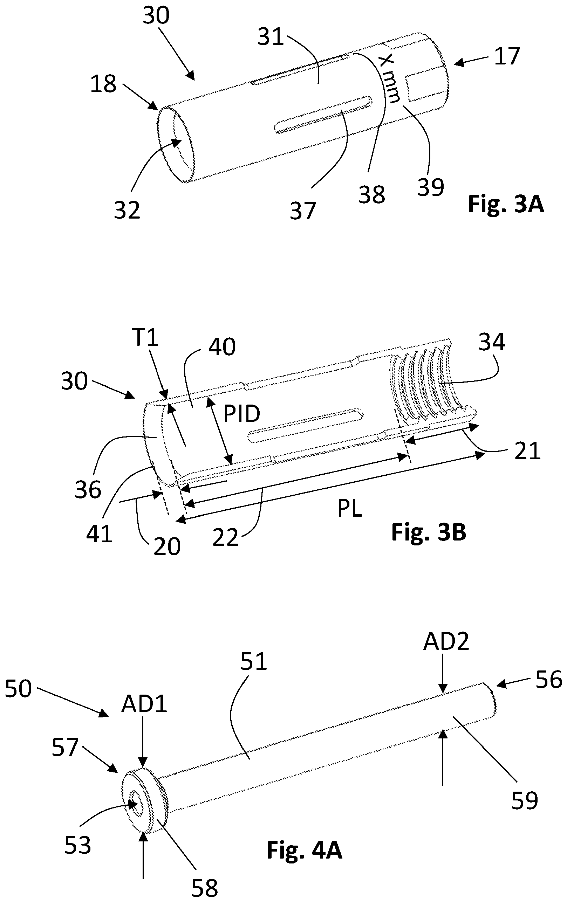

Figures 3A and 3B depict the punch head 30 (which may also be called a graft harvester) in a perspective view and a cross-sectional view, respectively. The punch head 30, which in this embodiment is a tubular and hollow element and has a substantially circular cross-section, comprises an elongated punch body 31 extending between a proximal end 17 (or an impaction handle coupling end) and a distal end 18, which also forms a punch cutting end. It is to be noted that the word tubular refers here to a tube-like shape, where the tube is a hollow and elongated body, but not necessarily having a circular cross section. It is to be noted that the punch head does not need to have a circular cross-section. The punch head may have a length of 15 mm - 35 mm while the largest cross-sectional diameter may be between 7 mm and 11 mm. A bore or channel 32 extends between the proximal end and the distal end. It is to be noted that in the present description, the word bore is understood to mean a hole, passage or tunnel irrespective of the manner the bore is created. For instance, the punch head could be made by three-dimensional printing without the need to create the bore by drilling. The proximal end 17 comprises in this embodiment a second coupling element, which is the second thread 34, which is shaped and sized to engage with the first thread 15 of the impaction handle 10. Thus, in this embodiment, the punch head is coupled with the impaction handle by means of a threaded connection. Instead of a threaded connection between the impaction handle and the punch head, other alternative coupling means may be envisaged, such as a snap connection or any other friction or form-fit connection. The punch head 30 has a punch head length PL. The punch head bore is delimited by a wall 40. At the distal end 18 of the punch head 30, there is provided a cutting edge 41 circumferentially around the bore and configured for bone cutting as explained later.

In this embodiment, the punch head 30 has a substantially cylindrical shape. The punch head can be divided longitudinally into three sections: a compression section 20 at the cutting end, a coupling element section 21 at the impaction handle coupling end, and a middle section 22 (or a thin-walled section) between the compression section and the coupling element section and having an inner diameter PID. The cross-sectional thickness of the middle section may be substantially constant. This thickness may be between 0.3 mm and 1.0 mm. The compression section comprises a compression surface 36 formed by a punch head bore center facing surface of the wall. The compression surface is circumferentially tapered (and has thus a beveled end) toward a center of the punch head bore such that the compression surface is arranged to apply a compression force on the bone plug such that the compression force increases while the bone plug advances toward the impaction handle coupling end inside the punch head bore. This force is directed substantially toward a central (longitudinal) axis of the bone plug while the bone plug advances toward the proximal end of the bone plug. The compression force may thus be substantially orthogonal to the surface of the wall of the punch head. The length of the compression section along the longitudinal axis of the punch head may be between 0.5 mm and 20 mm or between 0.5 mm and 10 mm or between 1 mm and 5 mm or more specifically between 2 mm and 5 mm. The wall thickness and the cutting edge largely define the bone penetration resistance. As shown in Figure 3B, in the compression section, the wall 40 is tapered toward the inner part of the punch head (its center) such that the cross-sectional thickness of the wall in the compression section gradually increases toward the proximal end of the punch head 30 (or the cross-sectional thickness of the wall gradually decreases toward the cutting edge 41) until it reaches a substantially constant thickness in the middle section. The punch head 30 shaped in this manner thus provides inwardly directed compression of the bone plug. However, it is to be noted that the compression surface could be designed so that the cutting edge is substantially flush with the inner wall of the middle section, and the compression surface would then taper toward the outside.

As is shown in Figure 3A, the punch head may also comprise indications to identify the punching depth, such as openings, slots or windows 37, lines 38, text (i.e. numbers and/or letters) 39. The openings also allow increasing the friction between the punch head and the bone plug so that the punch head is not allowed to rotate around the bone plug to allow it be broken off as explained later in more detail. The punch head may also include one or more ribs, such as longitudinal ribs, which may extend longitudinally along the inner wall of the punch head. Instead, or in addition, other protrusions or bumps may be provided on the inner wall. The purpose of these ribs or protrusions is also to increase friction between the bone plug and the punch head.

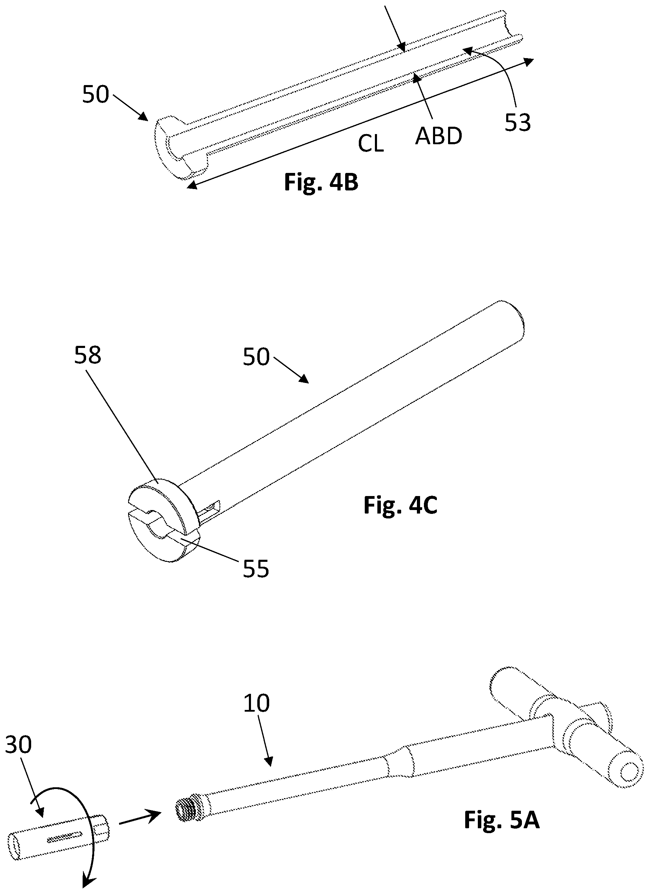

Figures 4A and 4B depict the centralization element 50 in a perspective view and a cross-sectional view, respectively. The centralization element 50 comprises an elongated centralization element body 51 extending between the proximal end 56 and the distal end 57 or a bone engaging end. As shown in Figures 4A and 4B, the elongated centralization element body 51 is a stepped body, having more than one external diameter. More specifically, the centralization element can be longitudinally divided into a first centralization element section 58 characterized by a first centralization element outer diameter AD1, and a second centralization element section 59 characterized by a second centralization element outer diameter AD2, which in this example is smaller than the first centralization element diameter. The first centralization element section is sized and shaped to be slidingly and in a form-fit manner received within the punch head bore and more specifically within its middle section, and the second centralization element section is sized and shaped to be at least partially slidingly and in a form-fit manner received within the cannulation 12 of the impaction handle and more specifically in its first cannulation section. Alternatively, the centralization element body has one single external diameter. The centralization element 50 has a length CL. In this embodiment the length CL is greater than the length PL. Moreover, the centralization element 50 comprises a bore or channel 53 extending between the proximal end 17 and the distal end 18 and thus the centralization element bore extends completely through the elongated centralization element body 51. The bore 53 has at least one bore diameter ABD. In the present embodiment, the bore 53 is sized and shaped to receive a guide wire 90 (shown in Figures 5A to 5H) in a substantially play free manner. Thus, the diameter ABD may substantially equal to the diameter CD2. Alternatively, the bore 53 may be shaped as a non-cylindrical channel. For manufacturing cost reasons, it is advantageous that the centralization element 50 is shaped rotationally symmetrical. Alternatively, the cross section of the centralization element may be of any other shape, such as a quadratic shape. The centralization element 50 is in the present embodiment designed so that it can be entirely received in a channel formed by the punch head bore 32 and the first cannulation section. Thus, a portion of the centralization element may also be received in the cannulation 12. As the periphery of the thickened portion of the centralization element is in this case designed to substantially mate with middle section of the punch head bore 32 or with its inner wall 40, the bore 53 is centrally aligned in the punch head bore 32.

Figure 4C depicts a variant of the centralization element wherein the first centralization element section 58 is minimally oversized in relation to the punch inner diameter PID and comprises at least one slot 55, which in this example is a longitudinal slot 55 extending from the distal end 57 toward the proximal end, providing elasticity and a friction fit when inserted into the punch head 30. The centralization instrument can in this manner sit in a stabile manner within the punch head proximal end when the surgeon brings the bone punch instrument in place. As soon as the surgeon starts impacting the punch head into the bone, the first centralization section will deflect inward, and slide toward the proximal end in a controlled manner.

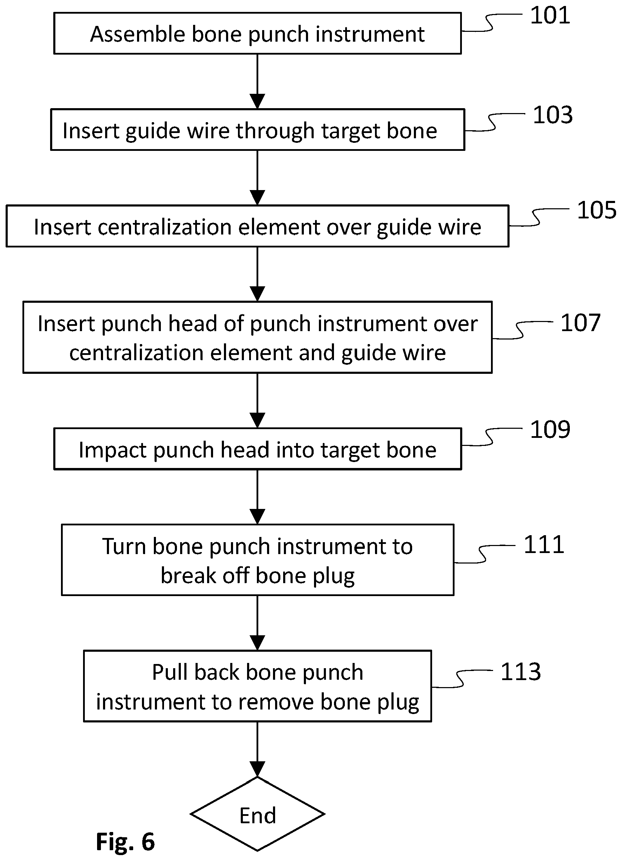

Referring to Figures 5A to 5H and to the flow chart of Figure 6, the femoral tunnel creation and the graft retrieval surgical steps are explained. For illustration purposes, tendons, ligaments, and the patella bone are blended out, providing a clear view of the tibial bone 60 and the femoral bone 61. It is to be noted that the method as described below may also be applied to other bones instead. In the present embodiment, the below process may be used as a part of an ACL reconstruction process.

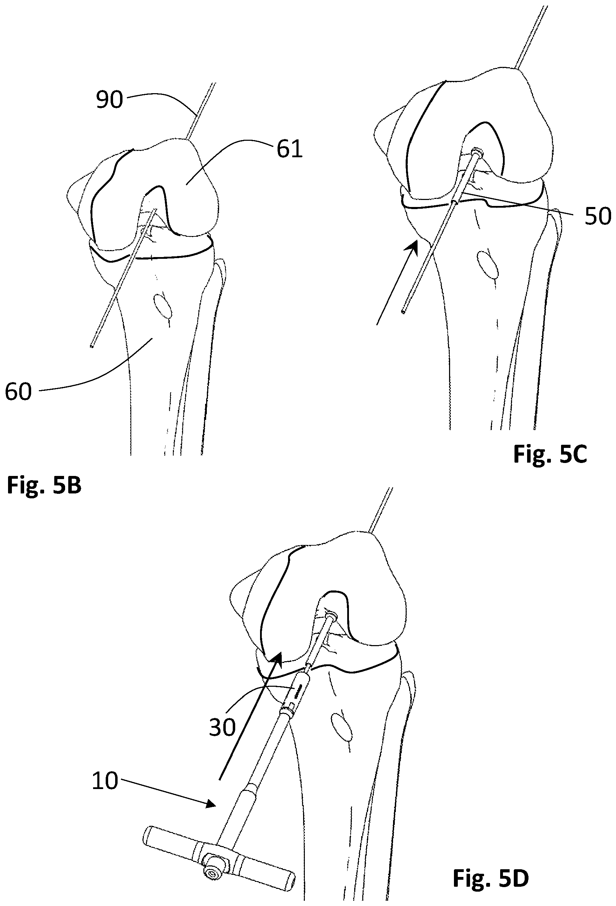

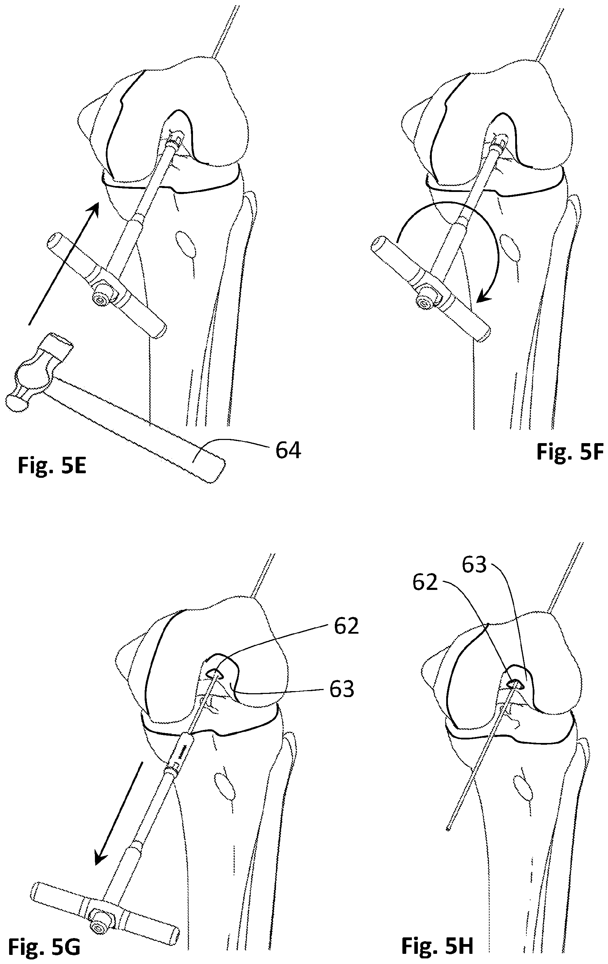

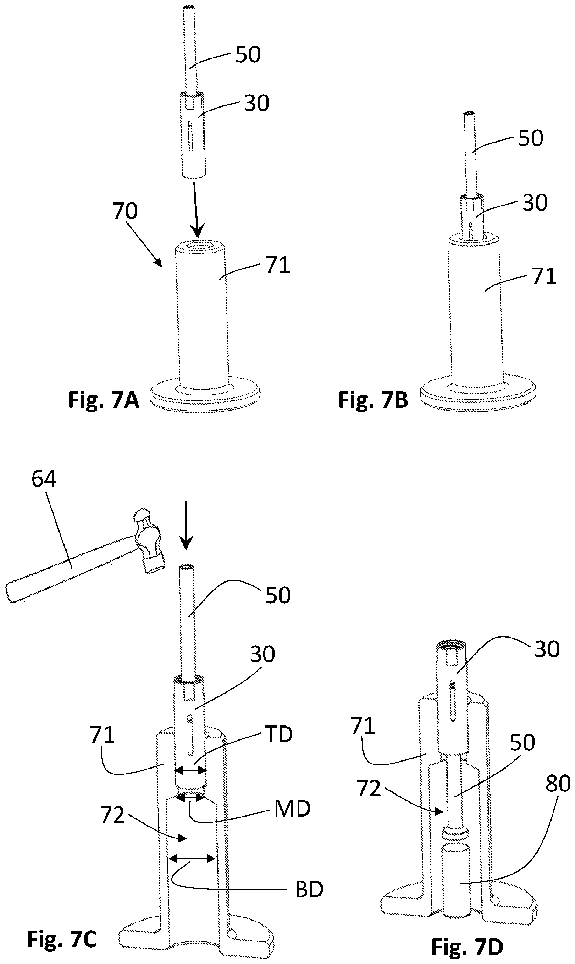

In step 101 and as shown in Figure 5A, the bone punch instrument 1 is assembled. More specifically, in this step the punch head 30 is connected to the impaction handle 10. In this specific example, the impaction handle is screwed into the punch head so that they are firmly coupled together. In step 103 and as shown in Figure 5B, a guide wire 90 is placed by drilling through the femoral bone 61 also referred to as a target bone. In step 105 and as shown in Figure 5C, the centralization element 50 is placed over the guide wire 90, and it is advanced forward until it comes in contact with the femoral bone 61. It is to be noted that this step is optional. In step 107 and as shown in Figure 5D, the punch head 30 when coupled with the impaction handle 10 is advanced forward over the guide wire 90 and the centralization element 50. The arrow in Figure 5D shows the direction of advancement. In step 109 and as shown in Figure 5E, the punch head 30 is forced into the target bone 61 and in this example into the inner side of the lateral femoral condyle also known as the lateral sidewall of the intercondylar notch, which may be considered to be part of the lateral femoral condyle or adjacent to the lateral femoral condyle. For this purpose there is provided a hammer or mallet 64, which is used by the operating person to hit or tap the proximal end of the impaction handle 10 to exert a force on the punch head. The direction of the force, which is toward the target bone, is shown in Figure 5E by a solid arrow and it substantially coincides with the longitudinal axis of the guide wire. The coupling between the impaction handle and the punch head is such that the force exerted on the force receiving end of the impaction handle is configured to be transferred onto the punch head. The guide wire 90 and the centralization element 50 facilitate guidance, and therefore all elements or instruments are centrally aligned with respect to each other. In this example, the punch head 30 is predominantly received in the target bone once impacted to the correct depth so that a small portion of the punch head still protrudes out of the target bone. In step 111 and as shown in Figure 5F, the operating person rotates the impaction handle. This rotation also causes the punch head 30 to turn in the same direction to break off the bone plug 80 (shown in Figure 7D). The impaction handle is turned clockwise (when seen from the operator) if a counterclockwise rotation is required to decouple the impaction handle from the punch head to break off the bone plug, and the impaction handle is turned counterclockwise if a clockwise rotation is required to decouple the impaction handle from the punch head to break off the bone plug. The turning thus takes places about the longitudinal axis of the shaft of the impaction handle and/or the punch head. Once the bone plug has been broken off, the bone plug remains in the punch head bore 32. In step 113 and as shown in Figure 5G, the bone plug 80 is removed out of the target bone by pulling back the impaction handle 10. As the punch head 30 is coupled with the impaction handle 10, also the punch head 30, the centralization element 50, and the bone plug 80 are pulled back together with the impaction handle 10. Figure 5H shows the created femoral tunnel 62, which in this case is a blind hole, at the inner side of the femoral condyle 63. It is to be noted that in the above process, step 101 may be carried out at any moment before carrying out step 107. The created bone tunnel typically has a length of 15 mm - 35 mm while the largest cross-sectional diameter is between 7 mm and 11 mm. It is to be noted that the punch head does not need to have a circular cross-section.

Referring to Figures 7A to 7D, a support instrument or punch head holder 70 is shown, which may be used to remove the bone plug from the punch head 30. The punch head holder may be considered to be part of the bone punch instrument or it can form an assembly or a system together with the bone punch instrument. However, it is to be noted that the punch head holder is an optional element that can be used to facilitate the removal of the bone plug out of the punch head but other suitable ways may be used instead for the bone plug removal. In this example, the punch head holder 70 has a tower-like body 71, and comprises a central stepped pocket or bore 72 at least partly extending longitudinally through the punch head holder. In this example, the pocket is a through bore inside the punch head holder. Furthermore, in this example, the stepped pocket has a cylindrical cross section, and has three diameters (although two or more diameters would equally be possible), namely a top diameter TD, a middle diameter MD and a bottom diameter BD. In this example, the top diameter TD is greater than the middle diameter MD, while the top diameter is smaller than bottom diameter BD. As shown in Figures 7A and 7B, the punch head 30 including the bone plug 80 and the centralization element 50 is placed in the punch head holder so that the punch head 30 is at least partially received in the pocket and more specifically in the upper pocket section having the top diameter. The middle diameter MD of the middle section of the punch head holder is smaller than the outer diameter of the punch head. In this manner, the middle section forms a stop for the punch head 30 and thus the punch head when inserted into the pocket from the top cannot advance beyond this stop. In fact, when in the pocket, the punch head 30 rests against the stop. The centralization element 50 extends out of the punch head 30 and more specifically out of its proximal end, and thus the centralization element forms a removal plunger for the bone plug 80. By means of for example tapping on the centralization element 50 and more specifically on its proximal end, the bone plug 80 is pressed out of the punch head 30, and captured in the punch head holder 70. The tapping may be executed by using the mallet 64. The bone plug may now be retrieved, and for example placed in the patella grafting site (not shown).

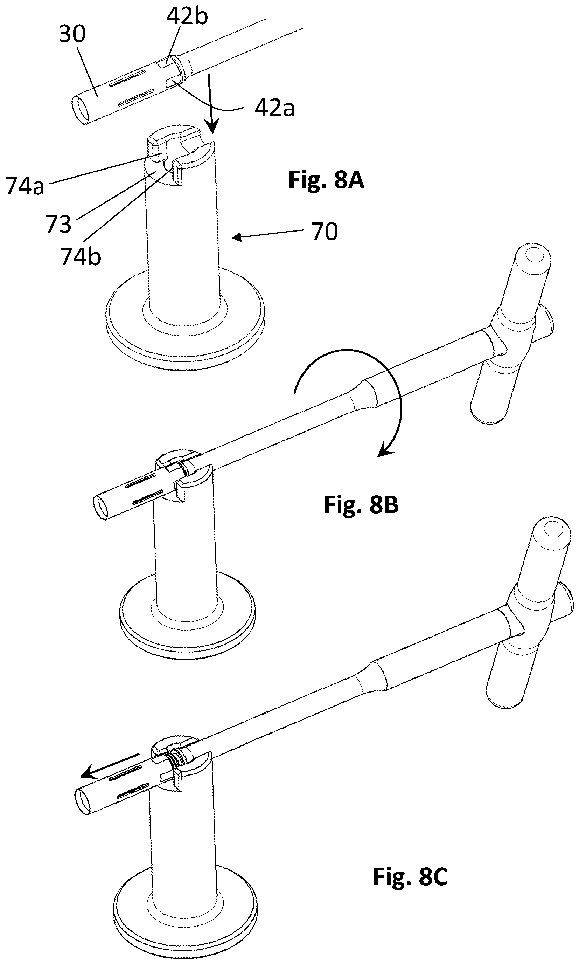

Figures 8A to 8C illustrate another embodiment of the invention. For ease of handling, the punch head 30 comprises at least one first key-face 42a, 42b forming a first (dis)assembly geometry and the punch head holder 70 defines a complementarily sized and shaped punch head receiving pocket 73 comprising at least one second key-face 74a, 74b forming a second (dis)assembly geometry. The faces 42a, 42b, 74a,74b in this example form a flat or a substantially flat surface. Upon insertion of the punch head into the receiving pocket, the faces of the punch head engage with the faces of the punch head holder 70, and a rotational degree of freedom between the punch head and the punch head holder is blocked. Now the impaction handle 10 can be disengaged from the punch head 30 by rotating the impaction handle. Hence, the punch head holder can be used as an disassembly aid. The integration of this kind of punch head holder eliminates the need of another instrument, and reduces surgery time.

The invention also proposes a femoral bone punch instrument set (or kit) comprising the femoral bone punch instrument as described above and further comprising instructions for using the set. The instructions may thus comprise instructions for carrying out one or more of the steps explained in the flow chart of Figure 6.

While the invention has been illustrated and described in detail in the drawings and foregoing description, such illustration and description are to be considered illustrative or exemplary and not restrictive, the invention being not limited to the disclosed embodiment. Other embodiments and variants are understood, and can be achieved by those skilled in the art when carrying out the claimed invention, based on a study of the drawings, the disclosure and the appended claims.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used.

## Claims

1. A femoral bone punch instrument (1) for creating a bone tunnel in a femoral condyle, the bone punch instrument (1) comprising:
• an impaction handle (10) having a force receiving end (17) and a punch head coupling end (18) comprising a first coupling element (15); and
• a hollow, elongated punch head (30) having a cutting end (18) and an impaction handle coupling end (17) comprising a second coupling element (34) configured to be removably coupled to the first coupling element (15) such that a force exerted on the force receiving end (17) of the impaction handle (10) is configured to be transferred onto the punch head (30), the punch head (30) comprising a punch head bore (32) for receiving a bone plug (80) of the femoral condyle, and a cutting edge (41) arranged circumferentially around the punch head bore (32) at the cutting end (18) of the punch head (30), the punch head (30) being sized and shaped to be forced into the femoral condyle to thereby circumferentially cut off the bone plug (80),
wherein the punch head bore (32) is delimited circumferentially by a wall (40), and **characterised in that**
the punch head (30) comprises at the cutting end (18) of the punch head (30) a compression surface (36) formed by a punch head bore facing surface of the wall (40), the compression surface (36) being circumferentially tapered toward a center of the punch head bore (32) such that the compression surface is arranged to apply a compression force on the bone plug (80) such that the compression force increases while the bone plug (80) advances toward the impaction handle coupling end (17) inside the punch head bore (32).

2. The bone punch instrument (1) according to claim 1, wherein the impaction handle (10) comprises a cannulation (12) extending longitudinally between the force receiving end (17) and the punch head coupling end (18).

3. The bone punch instrument (1) according to claim 2, wherein the cannulation (12) is a stepped cannulation having more than one diameter.

4. The bone punch instrument (1) according to claim 2 or 3, wherein the cannulation (12) is a stepped cannulation comprising a first cannulation section having a first cannulation diameter, and a second cannulation section having a second cannulation diameter.

5. The bone punch instrument (1) according to claim 4, wherein the first cannulation section extends from the punch head coupling end (18) end toward the force receiving end (17), while the second cannulation section extends from the a force receiving end (17) toward the punch head coupling end (18), and wherein the first cannulation diameter is greater than the second cannulation diameter.

6. The bone punch instrument (1) according to any one of claims 2 to 5, wherein the cannulation (12) extends longitudinally completely through the impaction handle (10).

7. The bone punch instrument (1) according to any one of the preceding claims, wherein the impaction handle (10) is a T-handle.

8. The bone punch instrument (1) according to any one of the preceding claims, wherein the wall (40) divided longitudinally along the length of the punch head (30) into a compression section (20) at the cutting end (18), a coupling element section (21) at the impaction handle coupling end (17), and a middle section (22) between the compression section (20) and the coupling element section (21), and wherein in the middle section (22) the wall has a cross-sectional thickness between 0.3 mm and 1.0 mm.

9. The bone punch instrument (1) according to any one of the preceding claims, wherein the first coupling element (15) is a male thread (15) and the second coupling element (34) is a female thread or vice versa, and wherein the male thread (15) is arranged to mate with the female thread (34) to form a threaded connection.

10. The bone punch instrument (1) according to any one of the preceding claims, wherein the punch head (30) has a largest cross-sectional diameter of 7 mm - 11 mm.

11. The bone punch instrument (1) according to any one of the preceding claims, wherein the punch head (30) has a length of 15 mm - 35 mm.

12. The bone punch instrument (1) according to any one of the preceding claims, wherein the punch head (30) is a cylindrical or substantially cylindrical element.

13. A bone punch instrument kit for creating a bone tunnel in a femoral condyle, the bone punch instrument kit comprising:
• an impaction handle (10) having a force receiving end (17) and a punch head coupling end (18) comprising a first coupling element (15); and
• a first elongated punch head (30) and a second hollow, elongated punch head (30), the first and second punch heads (30) comprising all features of the punch head of claim 1 and having a cutting end (18) and an impaction handle coupling end (17) comprising a second coupling element (34) configured to be removably coupled to the first coupling element (15) such that a force exerted on the force receiving end (17) of the impaction handle (10) is configured to be transferred onto the respective punch head (30), the first and second punch heads (30) comprising a punch head bore (32) for receiving a bone plug (80) of the femoral condyle, and a cutting edge (41) arranged circumferentially around the bore (32) at the cutting end (18) of the punch head (30), the first and second punch heads (30) being sized and shaped to be forced into the femoral condyle to thereby circumferentially cut off the bone plug (80), the first punch head (30) having a first length and a first punch bore diameter, and the second punch head having a second length and a second punch bore diameter, and wherein the first length is different from the second length and/or the first punch bore diameter is different from the second punch bore diameter.

## Patentansprüche

1. Femorales Knochenstanzinstrument (1) zum Erzeugen eines Knochentunnels in einem Femurkondylus, wobei das Knochenstanzinstrument (1) folgendes umfasst:
• einen Schlaggriff (10) mit einem Kraftaufnahmeende (17) und einem Stanzkopf-Kupplungsende (18), der ein erstes Kupplungselement (15) umfasst;
• einen hohlen, länglichen Stanzkopf (30) mit einem Schneidende (18) und einem Schlaggriff-Kupplungsende (17), der ein zweites Kupplungselement (34) umfasst, das so konfiguriert ist, dass es lösbar mit dem ersten Kupplungselement (15) gekoppelt werden kann, so dass eine auf das kraftaufnahmeende (17) des Schlaggriffs (10) ausgeübte Kraft konfiguriert ist auf den Stanzkopf (30) zu übertragen, wobei der Stanzkopf (30) eine Stanzkopfbohrung (32) zur Aufnahme eines Knochenstopfens (80) des Femurkondylus und eine Schneidkante (41) umfasst, die umlaufend um die Stanzkopfbohrung (32) am Schneidende (18) des Stanzkopfs (30) angeordnet ist, wobei der Stanzkopf (30) dimensioniert und geformt ist in den Femurkondylus gedrückt zu werden, um dadurch den Knochenstopfen (80) umlaufend abzuschneiden,
wobei die Stanzkopfbohrung (32) umfangsmäßig durch eine Wand (40) begrenzt ist, und dadurch gegenzeichnet, dass der Stanzkopf (30) am Schneidende (18) des Stanzkopfs eine Kompressionsfläche (36) umfasst, die durch eine der Stanzkopfbohrung zugewandte Oberfläche der Wand (40) gebildet wird, wobei die Kompressionsfläche (36) in Umfangsrichtung zur Mitte der Stanzkopfbohrung (32) hin verjüngt, so dass die Kompressionsfläche so angeordnet ist, dass sie eine Kompressionskraft auf den Knochenstopfen (80) ausübt, so dass die Kompressionskraft zunimmt, während sich der Knochenstopfen (80) in Richtung des Schlaggriff-Kupplungsende (17) innerhalb der Stanzkopfbohrung (32) bewegt.

2. Knochenstanzinstrument (1) nach Anspruch 1, wobei der Schlaggriff (10) eine Kanülierung (12) umfasst, die sich in Längsrichtung zwischen dem Kraftaufnahmeende (17) und dem Stanzkopf-Kupplungsende (18) erstreckt.

3. Knochenstanzinstrument (1) nach Anspruch 2, wobei die Kanülierung (12) eine abgestufte Kanülierung mit mehr als einem Durchmesser ist.

4. Knochenstanzinstrument (1) nach Anspruch 2 oder 3, wobei die Kanülierung (12) eine abgestufte Kanülierung mit einem ersten Kanülierungsabschnitt mit einem ersten Kanülierungsdurchmesser und einen zweiten Kanülierungsabschnitt mit einem zweiten Kanülierungsdurchmesser ist.

5. Knochenstanzinstrument (1) nach Anspruch 4, wobei der erste Kanülierungsabschnitt sich vom Stanzkopf-Kupplungsendes (18) zum Kraftaufnahmeende (17) erstreckt, während der zweite Kanülierungsabschnitt sich vom Kraftaufnahmeende (17) zum Stanzkopf-Kupplungsende (18) erstreckt, und wobei der erste Kanülierungsdurchmesser größer als der zweite Kanülierungsdurchmesser ist.

6. Knochenstanzinstrument (1) nach einem der Ansprüche 2 bis 5, wobei sich die Kanülierung (12) in Längsrichtung vollständig durch den Schlaggriff (10) erstreckt.

7. Knochenstanzinstrument (1) nach einem der vorhergehenden Ansprüche, wobei der Schlaggriff (10) ein T-Griff ist.

8. Knochenstanzinstrument (1) nach einem der vorhergehenden Ansprüche, wobei die Wand (40) in Längsrichtung entlang der Länge des Stanzkopfes (30) in einen Kompressionsabschnitt (20) am Schneidende (18), einen Kupplungselementabschnitt (21) am Schlaggriff-Kupplungsende (17) und einen Mittelabschnitt (22) zwischen dem Kompressionsabschnitt (20) und dem Kupplungselementabschnitt (21) unterteilt ist, und wobei im Mittelabschnitt (22) die Wand eine Querschnittsdicke zwischen 0.3 mm und 1.0 mm aufweist.

9. Knochenstanzinstrument (1) nach einem der vorhergehenden Ansprüche, wobei, das erste Kupplungselement (15) ein Außengewinde (15) und das zweite Kupplungselement (34) ein Innengewinde ist oder umgekehrt, und wobei das Außengewinde so angeordnet ist, dass es mit dem Innengewinde zusammenpasst, um eine Gewindeverbindung zu bilden.

10. Knochenstanzinstrument (1) nach einem der vorhergehenden Ansprüche, wobei der Stanzkopf (30) einen größten Querschnittsdurchmesser von 7 mm bis 1 mm aufweist.

11. Knochenstanzinstrument (1) nach einem der vorhergehenden Ansprüche, wobei der Stanzkopf (30) eine Länge von 15 mm bis 35 mm aufweist.

12. Knochenstanzinstrument (1) nach einem der vorhergehenden Ansprüche, wobei der Stanzkopf (30) ein zylindrisches oder im Wesentlichen zylindrisches Element ist.

13. Knochenstanzinstrumentensatz zum Erstellen eines Knochentunnels in einem Femurkondylus, wobei das Knochenstanzinstrumentensatz folgendes umfasst:
• einen Schlaggriff (10) mit einem Kraftaufnahmeende (17) und einem Stanzkopf-Kupplungsende (18), der ein erstes Kupplungselement (15) umfasst;
• einen ersten länglichen Stanzkopf (30) und einen zweiten hohlen, länglichen Stanzkopf (30), wobei der erste und der zweite Stanzkopf (30) alle Merkmale des Stanzkopfs nach Anspruch 1 umfassen, und wobei sie ein Schneidende (18) und ein Schlaggriff-Kupplungsende (17) aufweisen, das ein zweites Kupplungselement (34) umfasst, das so konfiguriert ist, dass es lösbar mit dem ersten Kupplungselement (15) gekoppelt werden kann so dass eine auf das kraftaufnahmeende (17) des Schlaggriffs (10) ausgeübte Kraft konfiguriert ist auf den jeweiligen Stanzkopf (30) zu übertragen, wobei der erste und der zweite Stanzkopf (30) eine Stanzkopfbohrung (32) zur Aufnahme eines Knochenstopfens (80) des Femurkondylus und eine Schneidkante (41) umfasst, die umlaufend um die Stanzkopfbohrung (32) am Schneidende (18) des Stanzkopfs (30) angeordnet ist, wobei der erste und der zweite Stanzkopf (30) so dimensioniert und geformt sind, dass sie in den Femurkondylus gedrückt werden, um dadurch den Knochenpfropfen (80) in Umfangsrichtung abzuschneiden, wobei der erste Stanzkopf (30) eine erste Länge und einen ersten Stanzbohrungsdurchmesser aufweist, und der zweite Stanzkopf eine zweite Länge und einen zweiten Stanzbohrungsdurchmesser aufweist, und wobei die erste Länge sich von der zweiten Länge unterscheidet und/oder der erste Stanzbohrungsdurchmesser sich vom zweiten Stanzbohrungsdurchmesser unterscheidet.

## Revendications

1. Un instrument de perforation osseuse fémorale (1) pour créer un tunnel osseux dans un condyle fémoral, l'instrument de perforation osseuse (1) comprenant :
• une poignée d'impaction (10) ayant une extrémité de réception de force (17) et une extrémité de couplage de tête de poinçon (18) comprenant un premier élément de couplage (15) ;
• une tête de poinçon creuse et allongée (30) comportant une extrémité de coupe (18) et une extrémité de couplage de poignée d'impaction (17) comprenant un second élément de couplage (34) configuré pour être couplé de manière amovible au premier élément de couplage (15) de telle sorte qu'une force exercée sur l'extrémité de réception de force (17) de la poignée d'impaction (10) est configurée pour être transférée sur la tête de poinçon (30), la tête de poinçon (30) comprenant un alésage de tête de poinçon (32) destiné à recevoir un bouchon osseux (80) du condyle fémoral, et un bord tranchant (41) disposé circonférentiellement autour de l'alésage de tête de poinçon (32) au niveau de l'extrémité de coupe (18) de la tête de poinçon (30), la tête de poinçon (30) étant dimensionnée et façonnée pour être forcée dans le condyle fémoral pour couper ainsi circonférentiellement le bouchon osseux (80),
dans lequel l'alésage de tête de poinçon (32) est délimité circonférentiellement par une paroi (40), et **caractérisé en ce que** la tête de poinçon (30) comprend à l'extrémité de coupe (18) de la tête de poinçon (30) une surface de compression (36) formée par une surface faisant face à l'alésage de tête de poinçon de la paroi (40), la surface de compression (36) étant circonférentiellement effilée vers un centre de l'alésage de tête de poinçon (32) de telle sorte que la surface de compression soit conçue pour appliquer une force de compression sur le bouchon osseux (80) de telle sorte que la force de compression augmente tandis que le bouchon osseux (80) avance vers l'extrémité de couplage de poignée d'impaction (17) à l'intérieur de l'alésage de tête de poinçon (32).

2. L'instrument de perforation osseuse (1) selon la revendication 1, dans lequel la poignée d'impaction (10) comprend une canulation (12) s'étendant longitudinalement entre l'extrémité de réception de force (17) et l'extrémité de couplage de tête de poinçon (18).

3. L'instrument de perforation osseuse (1) selon la revendication 2, dans lequel la canulation (12) est une canulation étagée ayant plus d'un diamètre.

4. L'instrument de perforation osseuse (1) selon la revendication 2 ou 3, dans lequel la canulation (12) est une canulation étagée comprenant une première section de canulation ayant un premier diamètre et une seconde section de canulation ayant un second diamètre.

5. L'instrument de perforation osseuse (1) selon la revendication 4, dans lequel la première section de canulation s'étend depuis l'extrémité de couplage de tête de poinçon (18) vers l'extrémité de réception de force (17), tandis que la seconde section de canulation s'étend depuis l'extrémité de réception de force (17) vers l'extrémité de couplage de tête de poinçon (18), et dans lequel le premier diamètre de canulation est supérieur au second diamètre de canulation.

6. L'instrument de perforation osseuse (1) selon l'une quelconque des revendications 2 à 5, dans lequel la canulation (12) s'étend longitudinalement complètement à travers la poignée d'impaction (10).

7. L'instrument de perforation osseuse (1) selon l'une quelconque des revendications précédentes, dans lequel la poignée d'impaction (10) est une poignée en T.

8. L'instrument de perforation osseuse (1) selon l'une quelconque des revendications précédentes, dans lequel la paroi (40) est divisée longitudinalement sur la longueur de la tête de poinçon (30) en une section de compression (20) au niveau de l'extrémité de coupe (18), une section d'élément de couplage au niveau de l'extrémité de couplage de poignée d'impaction (17), et une section médiane (22) entre la section de compression (20) et la section d'élément de couplage (21), et dans lequel, dans la section médiane (22), la paroi a une épaisseur transversale comprise entre 0.3 mm et 1.0 mm.

9. L'instrument de perforation osseuse (1) selon l'une quelconque des revendications précédentes, dans lequel le premier élément de couplage (15) est un filetage mâle (15) et le second élément de couplage (34) est un filetage femelle ou vice versa, et dans lequel le filetage mâle (15) est agencé pour s'accoupler avec le filetage femelle (34) pour former une connexion filetée.

10. L'instrument de perforation osseuse (1) selon l'une quelconque des revendications précédentes, dans lequel la tête de poinçon (30) a un diamètre maximal en coupe transversale de 7 mm à 11 mm.

11. L'instrument de perforation osseuse (1) selon l'une quelconque des revendications précédentes, dans lequel la tête de poinçon (30) a une longueur de 15 mm à 35 mm.

12. L'instrument de perforation osseuse (1) selon l'une quelconque des revendications précédentes, dans lequel la tête de poinçon (30) est un élément cylindrique ou un élément sensiblement cylindrique.

13. Un kit d'instruments de perforation osseuse pour créer un tunnel osseux dans un condyle fémoral, le kit d'instruments de perforation osseuse comprenant :
• une poignée d'impaction (10) ayant une extrémité de réception de force (17) et une extrémité de couplage de tête de poinçon (18) comprenant un premier élément de couplage (15) ;
• une première tête de poinçon allongée (30) et une seconde tête de poinçon creuse et allongée (30), la première tête de poinçon (30) et la seconde tête de poinçon (30) comprenant tous les éléments de la tête de poinçon de la revendication 1 et ayant une extrémité de coupe (18) et une extrémité de couplage de poignée d'impaction (17) comprenant un second élément de couplage (34) configuré pour être couplé de manière amovible au premier élément de couplage (15) de telle sorte qu'une force exercée sur l'extrémité de réception de force (17) de la poignée d'impaction (10) est configurée pour être transférée sur la tête de poinçon (30) respective, la première tête de poinçon (30) et la seconde tête de poinçon (30) comprenant un alésage de tête de poinçon (32) destiné à recevoir un bouchon osseux (80) du condyle fémoral, et un bord tranchant (41) disposé circonférentiellement autour de l'alésage (32) au niveau de l'extrémité de coupe (18) de la tête de poinçon (30), la première tête de poinçon (30) et la seconde tête de poinçon (30) étant dimensionnées et façonnées pour être forcées dans le condyle fémoral afin de couper ainsi circonférentiellement le bouchon osseux (80), la première tête de poinçon (30) ayant une première longueur et un premier diamètre d'alésage de poinçon, et la seconde tête de poinçon ayant une seconde longueur et un second diamètre d'alésage de poinçon, et dans lequel la première longueur est différente de la seconde longueur et/ou le premier diamètre d'alésage de poinçon est différent du second diamètre d'alésage de poinçon.
